# EUROPÄISCHE PATENTSCHRIFT

(11) **EP 3 305 227 B1**
(45) Veröffentlichungstag und Bekanntmachung des Hinweises auf die Patenterteilung: **16.03.2022**
(21) Anmeldenummer: 17001628.1
(22) Anmeldetag: 05.10.2017
(51) Int. Cl.: A61B 17/86

(54) **KNOCHENSCHRAUBE**
BONE SCREW
VIS À OS

(30) Priorität: 05.10.2016 DE 102016011947
(43) Veröffentlichungstag der Anmeldung: 11.04.2018
(73) Patentinhaber: Bluewater Medical GmbH, 24148 Kiel (DE)
(72) Erfinder: Lutz, Christian, 24226 Heikendorf (DE)
(74) Vertreter: Castell, Klaus

(56) Entgegenhaltungen:
- US-A1- 2012 101 534
- US-A1- 2013 131 733
- US-A1- 2016 235 447

## Beschreibung

Die Erfindung betrifft eine Schraube mit einem Kopfteil, einem Gewindeteil und einem flexiblen Verbindungsteil zwischen Kopfteil und Gewindeteil. Aufgabe der Schraube ist die Fixierung von zwei Knochen in einem festen Abstand zu einander, bei gleichzeitiger Erhaltung der Beweglichkeit der Knochen zu einander, beispielsweise zur Überbrückung eines Gelenkspaltes. Die Anforderung an die Schraube ist es möglichst hohe Zugkräfte aufnehmen zu können und dabei gleichzeitig einer Verschiebung der Knochen (quer zur Zugrichtung) einen möglichst geringen Widererstand entgegen zu setzten und dabei eine ausreichend hohes Drehmoment aufbringen zu können um in den Knochen hinein und auch wieder heraus geschraubt werden zu können.

Eine derartige Schraube ist aus der EP-A-1 273 269 MÜCKTER bekannt. Hier wird das flexible Verbindungsteil zwischen Kopfteil und Gewindeteil durch ein Drahtseil, eine Kordel, ein Drahtbündel oder durch Fäden gebildet. Das Drahtbündel kann durch eine von außen übergeschobene Hülse oder eine Spirale armiert sein. Bei der Implantation einer solchen Schraube wird zunächst der Gewindeteil mit einem Knochengewinde im Sinne eines Stehbolzens in den Knochen eingeschraubt. Dies geschieht mittels eines kanülierten Steckschlüssels, welcher über das Drahtseil oder das Drahtbündel bzw. die Kordel oder den Bolzen geschoben wird und am Außensechskant am Gewindeteil angreift. Danach wird eine Innensechskantkopfmutter mittels eines kanülierten Innensechskantschlüssels auf den Bolzen mit Metallgewinde aufgeschraubt. Abschließend wird das am Innensechskant überstehende Drahtseil oder Drahtbündel mit einer Kneifzange gekürzt. Die elastische Verbindung verleiht der Schraube zwar eine gewisse axiale Elastizität. Eine Übertragung von Drehmomenten ist jedoch ohne Zuhilfenahme des speziellen Steckschlüssels nicht möglich.

Aus der US 4,959,064 Engelhardt ist eine Knochenschraube mit einem Federteil bekannt. Der Federteil im Schaft der Knochenschraube verleiht ihr eine gewisse axiale Elastizität (axiale Kompression oder Distraktion). Eine Biegung ist möglich, eine Übertragung eines Drehmoments aber nicht.

Die Übertragung eines Drehmoments vom Kopfteil auf das Gewindeteil unter Beibehaltung einer flexiblen Verbindung beschreibt die EP-A-1 753 355 B1 NIEDERBERGER. Die Flexibilität zwischen Kopfteil und Gewindeteil wird durch ein Kugelgelenk erreicht, bei dem der Kopfteil eine Kugelschale und der Gewindeteil einen Kugelkopf aufweisen. Zur Übertragung eines Drehmoments hat der Kugelkopf einen achteckigen Querschnitt und die Kugelschale besitzt eine entsprechend angepasste, achteckige Geometrie, welche geeignet ist, den Querschnitt des Kugelkopfes aufzunehmen. Dadurch wird eine Rotation der Knochenschraube auch im abgewinkelten Zustand erzielt. Als Alternative wird auch ein Kreuz- oder Kardangelenk erwähnt.

Das Kugelgelenk mit achteckigem Kopf führt dazu, dass sich bei größeren Drehmomenten die acht Ecken am Kugelkopf abrunden und die Kugelschale aufweitet. Daher sind nur geringe Drehmomente übertragbar. Bei einem Kreuzgelenk ist die filigrane Kreuzgelenkverbindung für die Übertragung größerer Drehmomente und Zugkräfte mit kleinen Schaftdurchmessern nicht möglich.

Die WO 2006 105935 A1 erlaubt in einem Ausführungsbeispiel nur eine Knickbewegung und in den anderen Ausführungsbespielen werden zwar mehrere Knickbewegungen erlaubt. Dann ist aber die Schraube in ihrer Längserstreckung derart elastisch, dass sie es nicht mehr ermöglicht, den Abstand der Kochen zueinander präzise einhalten zu können und ein Drehmoment vom Kopfteil auf den Gewindeteil zu übertragen.

Die Erfindung betrifft insbesondere eine Schraube mit einem Kopfteil, einem Gewindeteil und einem flexiblen Verbindungsteil zwischen Kopfteil und Gewindeteil, um eine Torsionskraft zwischen Kopfteil, und Gewindeteil zu übertragen und in der Längsachse der Schraube zwei gegenläufige Knickbewegungen zwischen Kopfteil und Gewindeteil zu erlauben, wobei Kopfteil und Gewindeteil jeweils eine Längsachse aufweisen, die versetzt parallel zueinander anordenbar sind, und dass die Schraube bei einer Anordnung von Kopfteil und Gewindeteil auf einer Längsachse gegen eine Dehnung ein ε-Modul zwischen 20 000 und 100 000 N/mm² hat. Eine derartige Schraube ist aus der US 2013/131733 A1 bekannt.

Bei allen Ausführungsformen wird die Möglichkeit eines Versatzes zwischen Kopfteil und Gewindeteil mit einer unpräzisen Übertragung des Drehmoments zwischen Kopfteil und Gewindeteil erkauft.

Der Erfindung liegt daher die Aufgabe zu Grunde, derartige Schrauben dahingehend weiter zu entwickeln, dass sie für einen Einsatz zur Überbrückung auseinander liegender Knochenteile geeignet ist und die im Körper beispielsweise durch Bänder gegebene Stabilisierung unterstützt oder sogar vollständig übernimmt.

Diese Aufgabe wird bei einer gattungsgemäßen Schraube mit den Merkmalen des Patentanspruchs 1 gelöst. Die Übertragung besonders großer Zugkräfte bei begrenztem Schaftdurchmesser erschließt eine Vielzahl an Einsatzgebieten für eine derartige Schraube.

Vorteilhafte Weiterbildungen sind Gegenstand der Unteransprüche.

Der Erfindung liegt die Erkenntnis zu Grunde, dass es sinnvoll ist, für die Übertragung von Zugkräften und Drehmomenten unterschiedliche konstruktive Gestaltungen vorzusehen. Um diese für zwei unterschiedliche Funktionen jeweils optimierten Gestaltungen an einer gattungsgemäßen Schraube vorzusehen, werden Kupplung und flexible Verbindung konzentrisch zueinander angeordnet. Dies ermöglicht es, die aus dem Stand der Technik wie insbesondere der EP-A 1 273 269 MÜCKTER bekannten flexiblen Verbindungen zu verwenden und zu optimieren und andererseits eine Kupplung vorzusehen, die für die Übertragung eines Drehmoments optimiert ist. Die konzentrische Anordnung sorgt für einen kompakten Aufbau im Verbindungsbereich.

Die Kupplung kann als Seele im Zentrum des Verbindungsbereiches oder um eine Kanülierung herum angeordnet werden. Dann ist die flexible Verbindung radial außerhalb der Kupplung vorgesehen. Vorteilhaft ist es jedoch, die Kupplung um die flexible Verbindung herum anzuordnen. Insbesondere bei Drahtseilen, Kordeln, Drahtbündeln oder Fäden können diese innerhalb der Kupplung geführt und durch die Kupplung zusammengehalten werden. Auch ein Stab oder Rohr kann einfach innerhalb der Kupplung geführt und gehalten werden, um die axialen Kräfte aufzunehmen und eine flexible Verbindung zwischen Kopfteil und Gewindeteil zu ermöglichen. Die Torsionskräfte können dann im Wesentlichen von der Kupplung aufgenommen werden.

Folglich wird vorgeschlagen, dass bei einem Zug zwischen Kopfteil und Gewindeteil das flexible Verbindungsteil die Zugkräfte aufnimmt und dass bei einer Torsion zwischen Kopfteil und Gewindeteil die Kupplung die Torsionskräfte aufnimmt.

Die konstruktive Ausgestaltung ermöglicht es, als flexible Verbindung beispielsweise ein Seil, eine Litze oder eine Kette vorzusehen.

Im Hinblick auf die Verbindung zwischen Verbindungsteil und Kopfteil oder Gewindeteil wird vorgeschlagen, dass zumindest ein Teil des flexiblen Verbindungsteils im Kopfteil bzw. im Gewindeteil angeordnet ist. Dies ermöglicht eine Verbindung durch Schweißen, Löten, Schrumpfen oder Krimpen innerhalb vom Kopfteil oder vom Gewindeteil.

Um einen besonders kompakten Aufbau zu erzielen wird vorgeschlagen, dass die Kupplung formschlüssig mit Kopfteil und Gewindeteil verbunden ist. Dies ermöglicht es, bereits an der Verbindungsstelle zwischen Kopfteil und Gewindeteil eine gewisse Flexibilität zu erreichen.

Vorzugsweise weist die Kupplung mehrere Elemente auf, die formschlüssig miteinander verbunden sind. Dies ermöglicht es, auf einfache Art und Weise durch die Anzahl der Elemente die Flexibilität und die Länge des flexiblen Verbindungsteils zu verändern. Dabei sind die Elemente vorzugsweise gleichgeformt oder es wechseln sich zwei unterschiedlich geformte Elemente ab.

Eine bevorzugte Ausführungsform sieht vor, dass die Kupplung als formschlüssige Verbindung eine Nut- und Federverbindung aufweist. Dabei können an ringförmigen Elementen versetzt zueinander Nuten und Federn vorgesehen werden, damit beim hintereinander Anordnen mehrerer derartiger ringförmiger Elemente Nut und Feder ineinandergreifen.

Besonders bevorzugt ist es, als formschlüssige Verbindung an der Kupplung eine Schwalbenschwanzverbindung vorzusehen. Dies führt dazu, dass die einzelnen Kupplungselemente in axialer Richtung nicht auseinandergezogen werden können. Durch Auffädeln auf ein flexibles Verbindungsteil können die Elemente sogar unverlierbar angeordnet werden. Daher wird vorgeschlagen, dass die Kupplung mehrere ringförmige Elemente aufweist, die vorzugsweise auf dem flexiblen Verbindungsteil aufgefädelt sind.

Das Kopfteil kann einen gewindelosen Schaft und/oder ein Gewinde aufweisen. Entsprechend dem bekannten Funktionsprinzip der HERBERT-Schraube kann das Gewinde am Kopfteil gegenüber dem Gewindeteil einen größeren Durchmesser und eine kleinere Gewindesteigung aufweisen. Beim Eindrehen dieser Schraube in einen frakturierten Knochen senkrecht zur Frakturebene werden die beiden Fragmente aufeinander zu bewegt und gegeneinander verspannt, wobei sich das Maß der Zueinanderbewegung pro Schraubenumdrehung aus der Differenz der beiden Gewindesteigungen ergibt.

Unabhängig von der Ausbildung des Kopfteils ist es vorteilhaft, wenn der maximale Durchmesser von konzentrisch zueinander angeordnetem flexiblen Verbindungsteil und Kupplung nicht größer und vorzugsweise sogar kleiner ist als der maximale Außendurchmessers des Gewindeteils.

Derartige Schrauben sind für vielfältige Einsatzzwecke geeignet. Ein besonders relevanter Einsatzbereich liegt auf dem Bereich der Medizintechnik. Daher wird vorgeschlagen, dass die Schraube eine Knochenschraube ist.

Unterschiedliche Ausführungsbeispiele sind in der Zeichnung dargestellt und werden im Folgenden näher erläutert. Es zeigt:
- Figur 1: eine Ansicht einer Schraube mit Schwalbenschanzverzahnung,
- Figur 2: einen Schnitt durch die in Figur 1 gezeigte Schraube,
- Figur 3: eine perspektivische Ansicht der in Figur 1 gezeigten Schraube,
- Figuren 4 - 7: verschiedene Ansichten eines Kupplungselementes der in Figur 1 gezeigten Schraube,
- Figur 8: eine perspektivische Ansicht des Kupplungselementes der in Figur 1 gezeigten Schraube,
- Figur 9: einen Schnitt durch eine Schraube mit größerem Winkelspiel,
- Figur 10: eine erste Seitenansicht der in Figur 9 gezeigten Schraube,
- Figur 11: eine zweite Seitenansicht der in Figur 9 gezeigten Schraube,
- Figur 12: eine dreidimensionale Ansicht der in Figur 9 gezeigten Schraube,
- Figur 13: ein Kupplungselement mit Sternkörper,
- Figur 14: einen Schnitt durch zwei Mittels Klauen formschlüssig zusammenwirkenden Kupplungselementen,
- Figur 15: eine Ansicht eines Kupplungselementes gemäß Figur 14,
- Figur 16: eine dreidimensionale Ansicht von zwei zusammenwirkenden Kupplungselementen gemäß Figur 14,
- Figur 17: eine Schraube gemäß den Figuren 9 bis 12 in einer Ausrichtung mit versetzt parallel zum Gewindeteil angeordnetem Kopfteil und
- Figur 18: die in Figur 17 gezeigte Schraube eingesetzt zwischen Fibula und Tibia.

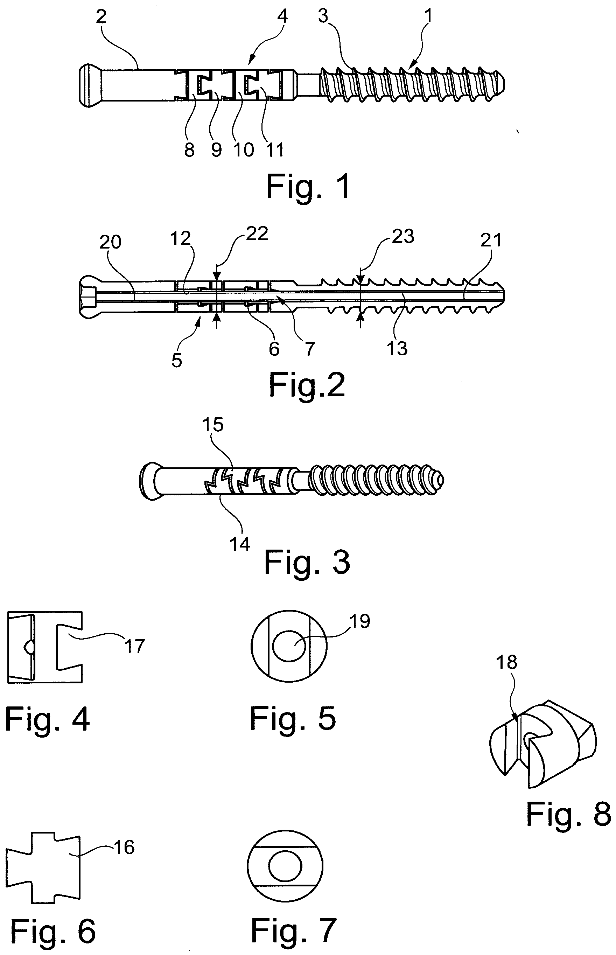

Die in Figur 1 gezeigte Schraube 1 hat einen Kopfteil 2 und ein Gewindeteil 3. Zwischen dem Kopfteil 2 und dem Gewindeteil 3 ist als flexibles Verbindungsteil 4 eine Kombination aus einem ersten Kupplungselement 5 mit einer Steckverbindung 6 zur Übertragung von Torsionskräften und einen zweiten Kupplungselement 7 zur Übertragung von Zugkräften vorgesehen. Das erste Kupplungselement 5 des flexiblen Verbindungsteils 4 besteht in axialer Richtung zwischen Kopfteil 2 und Gewindeteil 3 aus 4 hintereinander angeordneten Verbindungselementen 8, 9, 10 und 11.

Das erste Kupplungselement 5 mit den Verbindungselementen 8 bis 11, hat eine zentrale Bohrung 12 die es ermöglicht, als zweites Kupplungselement 7 einen flexiblen Draht innerhalb des ersten Kupplungselementes 5 anzuordnen. Dieser Draht 13 ist fest mit dem Kopfteil 2 und dem Gewindeteil 3 verbunden und erlaubt somit, die Übertragung von Zugkräften zwischen Kopfteil 2 und Gewindeteil 3 während der Draht Torsionskräften nur einen sehr geringen Widerstand entgegensetzt. Die Torsionskräfte werden über das erste Kupplungselement 5 vom Kopfteil auf das Gewindeteil über eine formschlüssige Verbindung 14 übertragen. Hierfür ist bei der Schraube 1 eine Nutfederverbindung 15 vorgesehen. Bei dieser Notfederverbindung ist die Feder 16 in der Nut 17 mit einem axialen Spiel von etwa 0,1 mm angeordnet. Im vorliegenden Fall summiert sich das Spiel zwischen den fünf Verbindungselementen, dem Kopfteil und dem Gewindeteil an den fünf Anlageflächen auf etwa 0,7 mm. Dies ermöglicht ein gewisses Winkelspiel, um die Längsachse der Schraube 1. Hierfür sind die Verbindungselemente 8 bis 11 ringförmig mit einer Schwalbenschwanzverbindung 18 ausgebildet, die quer zur Längsachse der Schraube ineinander gesteckt werden können und nach auffädeln auf den Draht 13 des zweiten Kupplungselementes 7 gegen verlieren und Verschiebungen gesichert sind. Hierfür weisen die einzelnen ringförmigen Verbindungselemente jeweils eine Bohrung 19 auf, in der als zweites Kupplungselement 7 an Stelle des in Figur 2 gezeigten flexiblen Drahtes auch ein Seil, eine Litze, eine Kette, ein Rohr, ein Stab oder eine Spirale angeordnet sein kann, sofern diese Elemente dazu geeignet sind, eine gewisse Torsionskraft zuzulassen und eine Zugkraft aufzunehmen.

Zur Befestigung des zweiten Kupplungselementes 7 im Kopfteil 2 ist das erste Ende 20 im Kopfteil 2 angeordnet und dort verschweißt. Während das zweite Ende 21 des zweiten Kupplungselementes 7 im Gewindeteil 3 verschweißt ist.

Der maximale Durchmesser 22 der Kupplungselemente 8 bis 11 ist etwas kleiner als der maximale Durchmesser 23 des Gewindeteils 3.

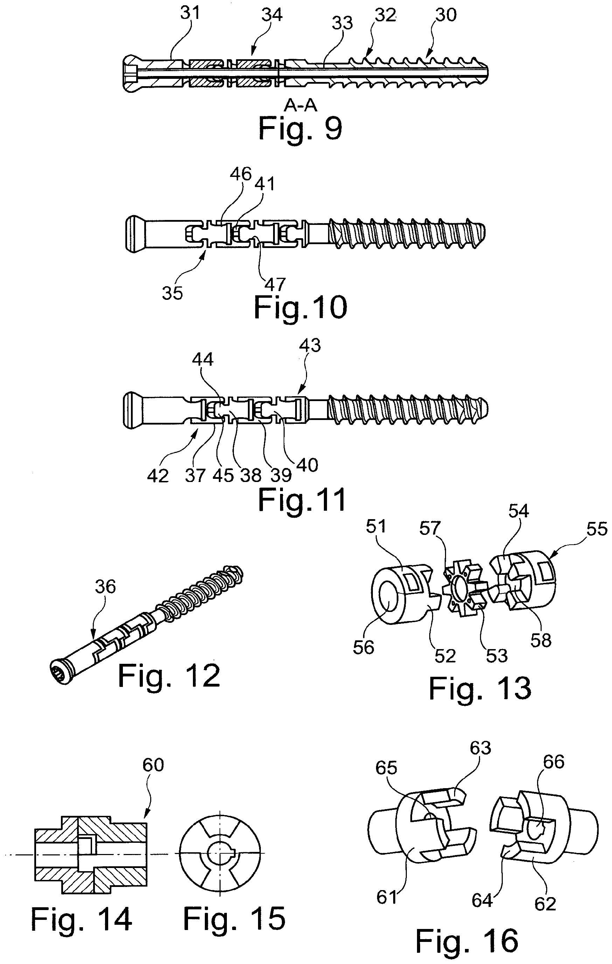

Die in den Figuren 9 bis 12 gezeigte Schraube 30 ist im Wesentlichen wie die Schraube 1 aus einem Kopfteil 31, einem Gewindeteil 32 und einem flexiblen Verbindungsteil 33 hergestellt. Die Schraube 30 unterscheidet sich jedoch von der Schraube 1 durch die Ausbildung des flexiblen Verbindungsteils 34. Das erste Kupplungselement 35 hat vier Verbindungselemente 37, 38, 39 und 40, die miteinander und mit dem Kopfteil 31 und dem Gewindeteil 32 über eine Steckverbindung 36 verbunden sind. Innerhalb diesen Verbindungselementen 37 bis 40 läuft als zweites Kupplungselement 41 der Draht.

Die Schraube 30 weist als formschlüssige Verbindung 42 eine spezielle Nutfederverbindung 43 auf, bei der ein Kopfteil 44 in einer Ansicht konvexe Anlageflächen 45 und in einer Ansicht senkrecht dazu konkave Anlageflächen 46 aufweist. Diese Anlageflächen wirken mit entsprechend geformten Anlageflächen einer Aufnahme 47 zusammen, die am selben Verbindungselement angeordnet ist.

Die spezielle Ausgestaltung der Verbindungselemente ermöglicht ein größeres Winkelspiel um die Längsachse der Schraube 30.

Neben der bei der Schraube 1 vorgesehenen Schwalbenschwanzverbindung 18 und der an der Schraube 30 vorgesehenen speziellen Nutfederverbindung 34, können auch andere formschlüssige Verbindungen vorgesehen werden. Die Figur 13 zeigt beispielsweise ein dreiteiliges Verbindungselement 50 aus einem ersten Halteteil 51, das mit Klauen 52 mit einem sternförmigen Mitteilteil 53 zusammenwirkt, während das sternförmige Mitteilteil 53 auch mit den Klauen 54 eines zweiten Halteteils 55 zusammenwirkt. Die Halteteile 51 und 55 sowie das sternförmige Mitteilteil 53 haben zentrale Bohrungen 56, 57 und 58, in denen ein flexibles Verbindungsteil (nicht gezeigt) geführt werden kann.

Die Figuren 14 bis 16 zeigen ein zweiteiliges Kupplungselement 60, bei dem Verbindungselemente 61 und 62 über Klauen 63 und 64 zusammenwirken, während beide Verbindungselemente jeweils eine zentrale Bohrung 65 und 66 aufweisen, in der ein zweites Kupplungselement (nicht gezeigt) geführt werden kann.

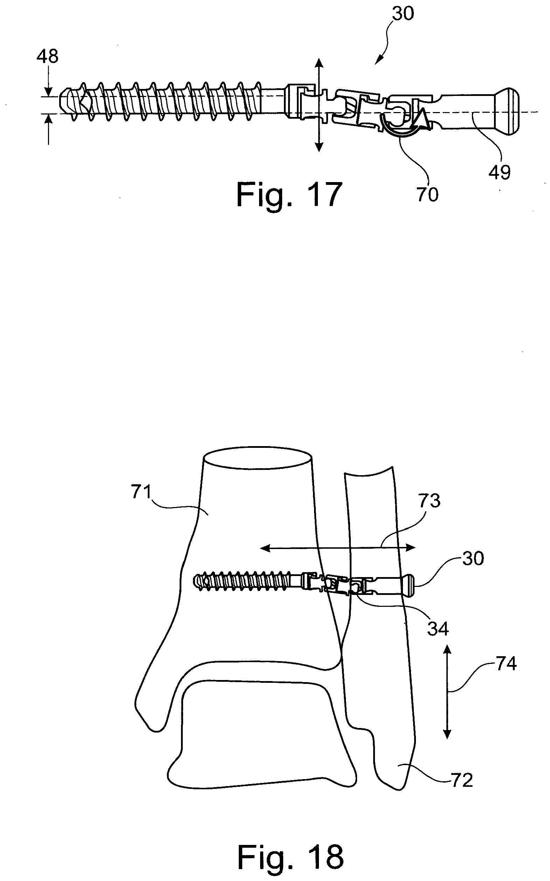

Die Figur 17 zeigt die in den Figuren 9 bis 12 gezeigte Schraube 30 mit einem Gewindeteil 32, das auf einer Längsachse 48 angeordnet ist, während das Kopfteil 31 auf einer parallel dazu versetzt liegenden Längsachse 49 angeordnet ist. Bei einer Rotation entsprechend dem Pfeil 70 dreht sich das Gewindeteil 32 ohne Spiel und ohne spürbare Elastizität mit dem Kopfteil 31.

Daher kann die Schraube 30 beispielsweise zwischen der Tibia 71 und der Fibula 72 eingesetzt werden, um die Fibula 72 entgegen in Z-Richtung 73 wirkenden hohen Kräften fest zu halten, während das flexible Verbindungsteil 34 in X- und Y-Richtung 74 eine sehr hohe Flexibilität erlaubt, um eine natürliche Bewegung des Gelenks zu ermöglichen. Dabei erstreckt sich die Z-Richtung in der Längsachse der Schraube und die X- und Y-Richtung erstrecken sich orthogonal dazu.

## Patentansprüche

1. Knochenschraube (1, 30) mit einem Kopfteil (2, 31), einem Gewindeteil (3, 32) und einem flexiblen Verbindungsteil (4, 34) zwischen Kopfteil (2, 31) und Gewindeteil (3, 32), um eine Torsionskraft zwischen Kopfteil (2, 31), und Gewindeteil (3, 32) zu übertragen und in der Längsachse der Schraube zwei gegenläufige Knickbewegungen zwischen Kopfteil (2, 31) und Gewindeteil (3, 32) zu erlauben, wobei Kopfteil (2, 31) und Gewindeteil (3, 32) jeweils eine Längsachse aufweisen, die versetzt parallel zueinander anordenbar sind, und dass die Schraube bei einer Anordnung von Kopfteil (2, 31) und Gewindeteil (3, 32) auf einer Längsachse gegen eine Dehnung ein ε-Modul zwischen 20 000 und 100 000 N/mm² hat, ***dadurch gekennzeichnet, dass*** das flexible Verbindungsteil (4, 33, 70) als Kupplung ein erstes Kupplungselement (5, 34, 50, 60) mit einer Steckverbindung (6, 36) zur Übertragung von Torsionskräften und ein zweites Kupplungselement (7, 41) zur Übertragung von Zugkräften aufweist.

2. Knochenschraube nach Anspruch 1, ***dadurch gekennzeichnet, dass*** die Schraube zwischen der Längsachse des Kopfteils und einer dazu parallelen Längsachse des Gewindeteils im Bereich des flexiblen Verbindungsteils einen Versatz von mindestens 0.3 mm pro 6 mm Länge des flexiblen Verbindungsteils aufweist.

3. Knochenschraube nach einem der vorhergehenden Ansprüche, ***dadurch gekennzeichnet, dass*** das flexible Verbindungsteil (4, 33) in axialer Richtung zwischen Kopfteil (2, 31) und Gewindeteil (3, 32) hintereinander mehrere zusammenwirkende Verbindungselemente (8 - 11, 37 - 40) aufweist.

4. Knochenschraube nach einem der vorhergehenden Ansprüche, ***dadurch gekennzeichnet, dass*** das erste (5) und das zweite Kupplungselement (7, 41) konzentrisch zueinander angeordnet sind.

5. Knochenschraube nach einem der vorhergehenden Ansprüche, ***dadurch gekennzeichnet, dass*** das erste Kupplungselement (5, 34) um das zweite Kupplungselement (7, 41) angeordnet ist.

6. Knochenschraube nach einem der vorhergehenden Ansprüche, ***dadurch gekennzeichnet, dass*** das erste Kupplungselement (5, 34) als formschlüssige Verbindung (14, 42) eine Nut-und- Feder-Verbindung (15, 43) aufweist.

7. Knochenschraube nach einem der vorhergehenden Ansprüche, ***dadurch gekennzeichnet, dass*** das erste Kupplungselement (5) als formschlüssige Verbindung (14) eine Schwalbenschwanzverbindung (18) aufweist.

8. Knochenschraube nach einem der vorhergehenden Ansprüche, ***dadurch gekennzeichnet, dass*** das erste Kupplungselement (5, 34) mehrere ringförmige Verbindungselemente (8 - 11, 37 - 40) aufweist.

9. Knochenschraube nach Anspruch 8, ***dadurch gekennzeichnet, dass*** die ringförmigen Verbindungselemente (8 - 11, 37 - 40) auf dem zweiten Kupplungselement (7, 41) aufgefädelt sind.

10. Knochenschraube nach einem der vorhergehenden Ansprüche, ***dadurch gekennzeichnet, dass*** das zweite Kupplungselement (7, 41) nur ein einziges Element mit nur einer zentralen Längserstreckungsachse ist.

11. Knochenschraube nach einem der vorhergehenden Ansprüche, ***dadurch gekennzeichnet, dass*** das zweite Kupplungselement (7, 41) ein Seil, eine Litze oder eine Kette ist.

12. Knochenschraube nach einem der vorhergehenden, ***dadurch gekennzeichnet, dass*** der Durchmesser des zweiten Kupplungselementes zwischen 0,5 und 5 mm beträgt.

13. Knochenschraube nach einem der vorhergehenden Ansprüche, ***dadurch gekennzeichnet, dass*** zumindest ein erstes Ende (20) des zweiten Kupplungselementes (7) im Kopfteil (2) angeordnet ist.

14. Knochenschraube nach einem der vorhergehenden Ansprüche, ***dadurch gekennzeichnet, dass*** zumindest ein zweites Ende (21) des zweiten Kupplungselementes (7) im Gewindeteil (3) angeordnet ist.

15. Knochenschraube nach einem der Ansprüche 8 und 9, ***dadurch gekennzeichnet, dass*** der maximale Durchmesser (22) der Kupplungselemente (8 - 11) nicht größer und vorzugsweise sogar kleiner ist als der maximale Außendurchmesser (23) des Gewindeteils (3).

16. Knochenschraube nach Anspruch 15, , ***dadurch gekennzeichnet, dass*** der Durchmesser (22) der Kupplungselemente (8 - 11) zwischen 2,0 mm und 8,5 mm beträgt.

## Claims

1. A bone screw (1, 30) comprising a head part (2, 31), a threaded part (3, 32), and a flexible connection part (4, 34) between head part (2, 31) and threaded part (3, 32), for transmitting a torsional force between head part (2, 31) and threaded part (3, 32), and for allowing two bending movements in the opposite direction between head part (2, 31) and threaded part (3, 32) in the longitudinal axis of the screw, wherein head part (2, 31) and threaded part (3, 32) in each case have a longitudinal axis, which can be arranged offset in parallel to one another, and that in the case of an arrangement of head part (2, 31) and threaded part (3, 32) on a longitudinal axis, the screw has an ε-module of between 20,000 and 100,000 N/mm² against an elongation, ***characterized in that*** the flexible connection part (4, 33, 70) as coupling has a first coupling element (5, 34, 50, 60) comprising a plug connection (6, 36) for transmitting torsional forces, and a second coupling element (7, 41) for transmitting tensile forces.

2. The bone screw according to claim 1, ***characterized in that*** the screw has an offset of at least 0.3 mm per 6 mm of length of the flexible connection part between the longitudinal axis of the head part and a longitudinal axis of the threaded part, which is parallel thereto, in the region of the flexible connection part.

3. The bone screw according to one of the preceding claims, ***characterized in that*** the flexible connection part (4, 33) has several cooperating connection elements (8 - 11, 37 - 40) one behind the other in the axial direction between head part (2, 31) and threaded part (3, 32).

4. The bone screw according to one of the preceding claims, ***characterized in that*** the first (5) and the second coupling element (7, 41) are arranged concentrically to one another.

5. The bone screw according to one of the preceding claims, ***characterized in that*** the first coupling element (5, 34) is arranged around the second coupling element (7, 41).

6. The bone screw according to one of the preceding claims, ***characterized in that*** the first coupling element (5, 34) has a tongue and groove connection (15, 43) as positive connection (14, 42).

7. The bone screw according to one of the preceding claims, ***characterized in that*** the first coupling element (5) has a dovetail connection (18) as positive connection (14).

8. The bone screw according to one of the preceding claims, ***characterized in that*** the first coupling element (5, 34) has several annular connection elements (8 - 11, 37 - 40).

9. The bone screw according to claim 8, ***characterized in that*** the annular connection elements (8 - 11, 37 - 40) are threaded onto the second coupling element (7, 41).

10. The bone screw according to one of the preceding claims, ***characterized in that*** the second coupling element (7, 41) is only a single element comprising only one central longitudinal axis of extension.

11. The bone screw according to one of the preceding claims, ***characterized in that*** the second coupling element (7, 41) is a rope, a cord, or a chain.

12. The bone screw according to one of the preceding claims, ***characterized in that*** the diameter of the second coupling element is between 0.5 and 5 mm.

13. The bone screw according to one of the preceding claims, ***characterized in that*** at least a first end (20) of the second coupling element (7) is arranged in the head part (2).

14. The bone screw according to one of the preceding claims, ***characterized in that*** at least a second end (21) of the second coupling element (7) is arranged in the threaded part (3).

15. The bone screw according to one of claims 8 and 9, ***characterized in that*** the maximum diameter (22) of the coupling elements (8 - 11) is not greater than and preferably even smaller than the maximum outer diameter (23) of the threaded part (3).

16. The bone screw according to claim 15, ***characterized in that*** the diameter (22) of the coupling elements (8 - 11) is between 2.0 mm and 8.5 mm.

## Revendications

1. Vis pour fracture osseuse (1, 30), pourvue d'une partie de tête (2, 31), d'une partie filetée (3, 32) et d'une partie de liaison (4, 34) souple entre la partie de tête (2, 31) et la partie filetée (3, 32), destinée à transmettre une force de torsion entre la partie de tête (2, 31) et la partie filetée (3, 32) et à permettre dans l'axe longitudinal de la vis deux mouvements d'angulation à contresens entre la partie de tête (2, 31) et la partie filetée (3, 32), la partie de tête (2, 31) et la partie filetée (3, 32) comportant chacune un axe longitudinal qui sont susceptibles d'être placés à la parallèle l'un de l'autre avec un décalage mutuel, et lors d'un placement de la partie de tête (2, 31) et de la partie filetée (3, 32) sur un axe longitudinal, la vis disposant d'un module élastique contre un allongement compris entre 20 000 et 100 000 N/mm², ***caractérisée en ce que*** la partie de liaison (4, 33, 70) souple comporte en tant qu'accouplement un premier élément d'accouplement (5, 34, 50, 60) doté d'une liaison enfichable (6, 36), pour transmettre des forces de torsion et un deuxième élément d'accouplement (7, 41) pour transmettre des forces de traction.

2. Vis pour fracture osseuse selon la revendication 1, ***caractérisée en ce qu***'entre l'axe longitudinal de la partie de tête et un axe longitudinal parallèle à celui-ci de la partie filetée, dans la zone de la partie de liaison souple, la vis présente un décalage d'au moins 0.3 mm par 6 mm de longueur de la partie de liaison souple.

3. Vis pour fracture osseuse selon l'une quelconque des revendications précédentes, ***caractérisée en ce que*** dans la direction axiale, entre la partie de tête (2, 31) et la partie filetée (3, 32), la partie de liaison (4, 33) souple comporte les uns derrières les autres plusieurs éléments de liaison (8 à 11, 37 à 40) qui sont en interaction.

4. Vis pour fracture osseuse selon l'une quelconque des revendications précédentes, ***caractérisée en ce que*** le premier (5) et le deuxième éléments d'accouplement (7, 41) sont placés de manière concentrique l'un par rapport à l'autre.

5. Vis pour fracture osseuse selon l'une quelconque des revendications précédentes, ***caractérisée en ce que*** le premier élément d'accouplement (5, 34) est placé autour du deuxième élément d'accouplement (7, 41).

6. Vis pour fracture osseuse selon l'une quelconque des revendications précédentes, ***caractérisée en ce que*** le premier élément d'accouplement (5, 34) comporte en tant que liaison (14, 42) par complémentarité de forme une liaison (15, 43) par rainure et languette.

7. Vis pour fracture osseuse selon l'une quelconque des revendications précédentes, ***caractérisée en ce que*** le premier élément d'accouplement (5) comporte en tant que liaison (14) par complémentarité de forme une liaison (18) en queue d'aronde.

8. Vis pour fracture osseuse selon l'une quelconque des revendications précédentes, ***caractérisée en ce que*** le premier élément d'accouplement (5, 34) comporte plusieurs éléments de liaison (8 à 11, 37 à 40) de forme annulaire.

9. Vis pour fracture osseuse selon la revendication 8, ***caractérisée en ce que*** les (éléments de liaison (8 à 11, 37 à 40) de forme annulaire sont enfilés sur le deuxième élément d'accouplement (7, 41).

10. Vis pour fracture osseuse selon l'une quelconque des revendications précédentes, ***caractérisée en ce que*** le deuxième élément d'accouplement (7, 41) n'est qu'un unique élément pourvu d'un seul axe central d'extension longitudinale.

11. Vis pour fracture osseuse selon l'une quelconque des revendications précédentes, ***caractérisée en ce que*** le deuxième élément d'accouplement (7, 41) est un câble, un toron ou une chaîne.

12. Vis pour fracture osseuse selon l'une quelconque des revendications précédentes, ***caractérisée en ce que*** le diamètre du deuxième élément d'accouplement se situe entre 0,5 et 5 mm.

13. Vis pour fracture osseuse selon l'une quelconque des revendications précédentes, ***caractérisée en ce qu***'au moins une première extrémité (20) du deuxième élément d'accouplement (7) est placée dans la partie de tête (2).

14. Vis pour fracture osseuse selon l'une quelconque des revendications précédentes, ***caractérisée en ce qu***'au moins une deuxième extrémité (21) du deuxième élément d'accouplement (7) est placée dans la partie filetée (3).

15. Vis pour fracture osseuse selon l'une quelconque des revendications 8 et 9, ***caractérisée en ce que*** le diamètre (22) maximal des éléments d'accouplement (8 à 11) n'est pas supérieur et de préférence, est même inférieur au diamètre extérieur (23) maximal de la partie filetée (3).

16. Vis pour fracture osseuse selon la revendication 15, ***caractérisée en ce que*** le diamètre (22) des éléments d'accouplement (8 à 11) se situe entre 2,0 mm et 8,5 mm.
